# EUROPEAN PATENT APPLICATION

(11) **EP 1 438 972 A1**
(43) Date of publication of application: **21.07.2004**
(21) Application number: 02762935.1
(22) Date of filing: 30.08.2002
(51) Int. Cl.: A61K 45/00, A61P 9/10, A61P 43/00

(54) **REMEDY FOR ISCHEMIC HEART FAILURE**

(30) Priority: 03.09.2001 JP 2001266489
(71) Applicant: CHUGAI SEIYAKU KABUSHIKI KAISHA, Tokyo, 115-8543 (JP)
(72) Inventor: SUZUKI, Yoshiyuki CHUGAI SEIYAKU KABUSHIKI KAISHA, Gotenba-shi, Shizuoka 41 2-8513 (JP); NAKANO, Kiyotaka CHUGAI SEIYAKU KABUSHIKI KAISHA, Gotenba-shi, Shizuoka 412 -8513 (JP); IMAGAWA, Jun-ichi CHUGAI SEIYAKU KABUSHIKI KAISHA, Gotenba-shi, Shizuoka 41 2-8513 (JP)
(74) Representative: VOSSIUS & PARTNER
(86) International application number: PCT/JP2002/008823
(87) International publication number: WO 2003/020312

(57) **Abstract**

In the process of extensively searching for a drug elevating the survival ratio in ischemic heart failure, in particular postinfarction heart failure, it is found out that βARKct which is a βARK1 inhibitor is efficacious not only in improving heart function but also elevating survival ratio in postinfarction heart failure. Accordingly, it is expected that the βARK1 inhibitor is efficaciously usable as a remedy for ischemic heart failure which exerts not only an effect of improving heart function but also another effect of elevating survival ratio and, in its turn, contributes to the improved prognosis in patients with ischemic heart diseases.

## Description

### Technical Field

This invention relates to inhibitors for β-adrenergic receptor kinase 1, which comprise an effect of improving the survival rate in ischemic heart failure, and therapeutic agents for ischemic heart failure, which comprise an inhibitor for β-adrenergic receptor kinase 1 as an active ingredient.

### Background Art

Chronic heart failure is one of the cardiovascular diseases with extremely poor prognosis, and its five year survival rate is estimated to be 50% or less. Chronic heart failure is the final condition of a variety of diseases. This disease is categorized as ischemic heart failure or non-ischemic heart failure (mainly dilated cardiomyopathy) based on the underlying disease. Ischemic heart failure and non-ischemic heart failure have been indicated to have different pathology from each other. For example, cardiac insufficiency bisoprolol study (CIBIS) test has been reported to reveal that bisoprolol reduces the death rate of the non-ischemic heart failure patients by 37%, while no improvement in the death rate is found in the patients with ischemic heart failure (CIBIS Investigators and Committees, Circulation 90: 1765-1773, 1994). Furthermore, prospective randomized amlodipine survival evaluation (PRAISE) test revealed that amlodipine reduces the death rate of the non-ischemic heart failure patients by 46%, while no improvement in the death rate is found for the ischemic heart failure patients (Packer M *et al.*, N Engl J Med 335: 1107-1114, 1996). These results indicate the pathology is different between ischemic heart failure and non-ischemic heart failure.

Patients with ischemic heart failure, in particular with heart failure after myocardial infarction, have remarkably increased in recent years especially in Europe and in North America. This increase has been caused by the significant decrease in the death rate brought by the progress of myocardial infarction treatments (Chien KR. Cell 98: 555-558, 1999). The results of recent large scale clinical studies indicate that ischemic heart failure has still poor prognosis while angiotensin converting enzyme (ACE) inhibitors is the first-line drug for this disease. Various drugs are currently under evaluation and β-blockers which enhance the survival rate have been indicated to aggravate the heart failure conditions at the beginning of their administration in more than a few cases. In addition, cardiotonics which improve a short-term quality of life (QOL) have been shown to worsen the long-term QOL (Packer M *et al.*, N Engl J Med 325: 1468-1475, 1991).

β-adrenergic receptor kinase 1 (βARK1) is one major molecule that regulates the signal transduction pathway of β-adrenergic receptor (βAR) expressed in cardiomyocytes. It is indicated that βARK1 phosphorylates βAR and induces desensitization of the βAR. Amino acid and DNA sequences of βARK1 have already been revealed for a bovine (Benovic J.L. *et al.*, Science 246: 235-240, 1989), hamster (Urasawa K. *et al.*, Biochem Biophys Res Commun 219: 26-30, 1996), rat (Arriza J. L. *et al.*, J Neurosci 12: 4045-4055, 1992), human (Benovic J. L. *etal.*, FEBS Lett 283: 122-126, 1991), and such, and βARK1 can be produced by genetic engineering technique. Separation and purification procedures for a natural βARK1 protein have also been established. Furthermore, substrates for βARK1 have been found and βARK1 activity can be determined by established methods (Benovic J. L. Methods Enzymol 200: 351-362, 1991; Pitcher J. A. *et al.* Annu Rev Biochem 67: 653-692, 1998).

Currently known chronic heart failure models include, based on the underlying diseases, hypertensive heart failure models (rat and mouse models of aortic banding, Dahl salt-sensitive (DS) rat model, spontaneously hypertensive rat with heart failure (SHHF), and such) ; ischemic heart failure models (rat and mouse models of heart failure after myocardial infarction, and so on); and dilated cardiomyopathy (DCM) models (cardiomyopathic hamster, rabbit and dog pacing models; genetically modified animal models (muscle LIM protein knockout (MLP KO) mouse, calsequestrin transgenic (CSQ TG) mouse, and such) etc.).

It has been recently reported that, as heart failure progressed, βAR responsiveness was impaired with enhanced expression and activity of βARK1 in dilated cardiomyopathy (DCM) models (CSQ TG mice) (Cho MC *et al.*, J Biol Chem 274: 22251-22256, 1999). Furthermore, in CSQ TG mice, a βARK1 inhibitor, βARKct (a peptide comprising the amino acids of position 495 to 689) was indicated to improve the cardiac function as well as the survival rate (Harding VB *et al.*, Proc Natl Acad Sci USA 98: 5809-5814, 2001). In addition, it was reported that the cardiac function was improved by introducing βARKct into the cardiac muscle of rabbit models of heart failure after myocardial infarction (White DC *et al.*, Proc Natl Acad Sci USA 97: 5428-5433, 2000; Shah AS *et al.*, Circulation 103: 1311-1316, 2001). However, it remains to be revealed whether a βARK1 inhibitor improves the survival rate.

These results indicate that a βARK1 inhibitor has the effects of improving cardiac function and improving the survival rate in non-ischemic heart failures, as well as the cardiac function-improving effect in ischemic heart failure. However, no reports so far have showed that a βARK1 inhibitor has the survival rate-improving effect in ischemic heart failure.

### Disclosure of the Invention

The present invention has been made under circumstances as described above. An objective of the present invention is to clarify that βARK1 inhibitors comprise an effect of improving the survival rate in ischemic heart failure, and to provide βARK1 inhibitors which comprise an effect of improving the survival rate in ischemic heart failure, as well as therapeutic agents for ischemic heart failure, which comprise a βARK1 inhibitor as an active ingredient.

In the process of exhaustively searching for drugs which elevate the survival rate in ischemic heart failure, in particular heart failure after myocardial infarction, the present inventors found that βARKct, which is a βARK1 inhibitor (Koch WJ *et al.*, Science 268: 1350-1353, 1995), improves not only cardiac function but also the survival rate in heart failure after myocardial infarction. Specifically, when transgenic mice comprising βARKct and control mice were operated to produce myocardial infarction, the transgenic mice exhibited a marked recovery in the cardiac function compared to the control mice. The transgenic mice also showed a significantly enhanced survival rate after the surgery compared to the control mice. Accordingly, it is highly expected that βARK1 inhibitors would serve as an effective therapeutic agent for ischemic heart failure, which can improve the survival rate as well as cardiac function.

This invention relates to βARK1 inhibitors comprising a survival rate-improving effect in ischemic heart failure, and therapeutic agents for ischemic heart failure, which comprise a βARK1 inhibitor as an active ingredient. More specifically, the present invention provides:
[1] an inhibitor for β adrenergic receptor kinase 1 (βARK1) which comprises an effect of improving the survival rate in ischemic heart failure;
[2] a therapeutic agent for ischemic heart failure, which comprises a βARK1 inhibitor as an active ingredient and comprises an effect of improving the survival rate in ischemic heart failure;
[3] the therapeutic agent according to [2], wherein the ischemic heart failure is heart failure after myocardial infarction; and
[4] a method for improving the survival rate of a patient with ischemic heart failure, comprising administering to the patient the inhibitor of [1] or the therapeutic agent of [2] or [3] in an amount effective to improve the cardiac function of the patient.

The present inventors have discovered for the first time that βARK1 inhibitors comprise a survival rate-improving effect in ischemic heart failure. Accordingly, it is highly expected that βARK1 inhibitors would serve as therapeutic agents for ischemic heart failure, which comprise a survival rate-improving effect. The present invention provides βARK1 inhibitors comprising a survival rate-improving effect in ischemic heart failure, and therapeutic agents for ischemic heart failure, which comprises a βARK1 inhibitor as an active ingredient.

The term "heart failure" as used herein means the condition in which blood is not ejected in an amount necessary for the tissue metabolism of the entire body due to an impaired cardiac function, or the condition in which such ejection of a necessary amount of blood is only enabled by an increased ventricular filling pressure. The expression "ischemic heart failure" refers to pathology showing chronic heart failure with the dilated left ventricle and reduced contractility. This disease is caused by wide myocardial necrosis induced by myocardial infarction, or by a myocardial disease accompanied with serious chronic myocardial ischemia. A typical ischemic heart failure to be treated by the present invention is, not limited to, but heart failure after myocardial infarction. The expression "myocardial infarction" means myocardial necrosis of the size visible to naked eye. Such necrosis is caused by a certain time period of continuous myocardial ischemia induced by the interruption of the circulation due to the obstruction or stenosis of a coronary artery. Myocardial infarction is generally complicated with arrhythmia, heart failure, and so on. Heart failure is a frequently observed pathology secondary tomyocardial infarction, and especially, congestive heart failure is found in 20 to 50% of myocardial infarction patients. The heart failure that is induced secondarily by the primary myocardial infarction is called "heart failure after myocardial infarction". Congestive heart failure is a typical heart failure and is included in heart failure after myocardial infarction.

Several pharmaceutical treatments are known for heart failures including congestive heart failure. For example, diuretics can reduce preload and are expected to be effective for pulmonary congestion or systemic edema. Angiotensin converting enzyme (ACE) inhibitors suppress renin-angiotensin-aldosterone system and have an effect of reducing preload and afterload and an effect of suppressing stimulation by angiotensin II. Specifically, ACE inhibitors act as vasodilators on both venous and arterial systems to dilate the blood vessels, and suppress the effects of neural and humoral factors that have been activated by heart failure, such as angiotensin II, aldosterone, norepinephrine, vasopressin, and other neurohumoral factors. ACE inhibitors may also suppress the mechanisms of myocardial remodeling to prevent the onset of heart failure after myocardial infarction. β blockers have been reported to be effective for dilated cardiomyopathy by their effects of reducing the contractility and suppressing the excitation of sympathetic nerves. Thus, β blockers are expected to improve the QOL and prognosis. Various vasodilators effective for reducing afterload can also be used as therapeutic agents for heart failures. Drugs known to act on the venous system include nitrites and molsidomine, and drugs known to have the effect on the arterial system include hydralazine, minoxidil, and calcium antagonists. Flosequinan and nitroprusside are known to act on both the venous and the arterial systems. Digitalis is a cardiotonic and has been reported to exert its effect through its functions to increase contractility (positive inotropic action), to reduce heart rate (negative chronotropic action), to suppress a sympathetic efferent pathway, and to improve baroreflex sensitivity.

The expression "effect of improving the survival rate" as used herein means an effect such that the survival rate of patients who have received administration of a pharmaceutical agent is significantly higher than that of patients without administration of the pharmaceutical agent. Alternatively, the expression refers to an effect such that the patients who have received administration of a pharmaceutical agent survive longer than the patients without administration of that pharmaceutical agent. In the present invention, a survival rate-improving effect may be measured by any methods known to one skilled in the art. For example, this effect may be determined by a clinical method. In such a method, the effect can be determined by observation of a survival curve obtained after administration of a therapeutic agent (herein also referred to as a pharmaceutical agent). Specifically, the patients are divided into a group with administration of a pharmaceutical agent (administered group) and a group without administration (control group), and survival and death of each patient are monitored in the course of time. More specifically, the survival curve may be produced by Kaplan-Meier method from the cumulative survival rate, and the significant difference may be examined by log-rank test. Such analyses may be conducted by using a commercially available analysis software such as StatView ver.5 for Windows. For example, the survival rate of patients may be calculated by confirming survival or death of each patient after a certain period of time. A survival curve may be obtained for each of the administered group and the control group by taking the survival rate at the start of the administration as 100% or 1, and by plotting the cumulative survival rate on the vertical axis and the time on the horizontal axis. If comparison of the survival curves between the administered group and the control group shows that the survival curve of the administered group is plotted upper than that of the control group, the pharmaceutical agent that has been administered can be determined to have a survival rate-improving effect. Alternatively, the survival rate of these two groups may be compared after a certain period. When the survival rate of the administered group is significantly higher than that of the control group, the pharmaceutical agent can be judged to comprise a survival rate-improving effect. Such a certain period may be at least half a year, preferably one year, more preferably two years, and most preferably three years. It is not necessary to track the control group simultaneously with the administered group, and the survival rate and the survival curve of the patients that have been previously investigated may be used as those of the control. More specifically, when the survival curve of the administered group is plotted significantly upper than that of the control, the pharmaceutical agent may be determined to comprise a survival rate-improving effect.

A survival rate-improving effect can also be confirmed by using experimental animals. In this case, the effect may be determined by using a disease model animal and comparing the survival curves between the pharmaceutical agent-administered animals and the animals without administration. More specifically, when the survival curve of the administered animals is plotted upper than that of animals without the administration, the pharmaceutical agent can be judged to comprise a survival rate-improving effect. Examples of disease model animals mentioned above include, but are not limited to, ischemic heart failure models (mouse, rat, rabbit, dog, and pig models of heart failure after myocardial infarction) (Hongo M. *et al.*, Trends Cardiovasc Med 7: 161-167, 1997; Patten R. D. *et al.,* Am J Physiol 274: H1812-H1820, 1998).

βARK1 inhibitors of the present invention comprising a survival rate-improving effect in ischemic heart failure may also have other effects. Such other effects include a cardiac function-improving effect. Accordingly, a βARK1 inhibitor comprising a survival rate-improving effect as well as a cardiac function-improving effect is encompassed in βARK1 inhibitors of the present invention comprising a survival rate-improving effect in ischemic heart failure.

The improved heart function may be determined by a method generally used in the art, for example, the New York Heart Association (NYHA) functional classification or echocardiography. NYHA has proposed the following four functional classes of heart functions to evaluate heart functions of heart failure patients.
(1) Class I: Patients with cardiac disease without limitation of physical activities. Ordinary activities do not cause symptoms such as dyspnea, angina, fatigue, or palpitation.
(2) Class II: Patients with cardiac disease with slight limitation of physical activities. They are comfortable with rest or mild exertion. Relatively hard ordinary activities cause symptoms as described above.
(3) Class III: Patients with cardiac disease with marked limitation of physical activities. They are comfortable only at rest. Less than ordinary activity causes symptoms as described above.
(4) Class IV: Patients with cardiac disease, who cannot carry on any physical activity without symptoms as described above. Heart failure or anginal syndrome may occur even at rest and progressed by physical activities.

Of these classes, class I patients do not need pharmacotherapy and can be regarded as being at a normal level. Accordingly, when a patient who has been in class II, class III, or class IV is shifted into class I, the heart function of the patient can be judged to have been improved. Measured values of heart function determined using echocardiography include left ventricular ejection fraction (EF) and left ventricular fractional shortening (FS). The normal value for EF is between 60% and 80%, and the normal value for FS is between 30% and 50%. Heart failure patients exhibit clearly lower EF or FS than the normal values.

As described above, the expression "an effect of improving heart function" refers to a decrease in the class number of the NYHA functional classification and/or the increase in EF or FS, which is low in a patient with heart failure. While both of EF and FS are preferably improved to the degree within the normal values, it is still acceptable that either one of them is improved to the normal level. Accordingly, in the present invention, a pharmaceutical agent comprising a cardiac function-improving effect may be determined to enable a decrease in the class number of the NYHA functional classification and/or an increase in the EF or ES value which is low in heart failure patients.

A cardiac function-improving effect of a pharmaceutical agent may also be determined using experimental animals. In this case, systolic ejection function (FS or EF) may be measured by echocardiography. Heart failure animal models such as DS rat models, rat or mouse models of heart failure after myocardial infarction, and DCM models (e.g., a cardiomyopathic hamster, a rabbit or dog pacing model, a genetically modified animal model (MLP KO mouse, CSQ TG mouse, and such)), have clearly lower FS and EF values than that of normal animals. In the present invention, pharmaceutical agents which statistically significantly increase the lowered FS or EF are determined to comprise a cardiac function-improving effect.

βARK1 inhibitors of the present invention are not particularly limited as long as they comprise an effect of inhibiting or suppressing the function of βARK1. The βARK1 inhibitors may be nucleic acids, proteins, peptides, or such. Exemplary proteins and peptides include a dominant negative of βARK1 and a peptide fragment of βAR. Exemplary nucleic acids include a vector into which a DNA encoding a peptide or a protein comprising amino acids such as the dominant negatives as mentioned above.

A dominant negative of βARK1 described above means a protein or a peptide which lacks the function necessary to exert the effects of βARK1. Such a dominant negative of βARK1 may typically include a peptide fragment of βARK1. Functions deficient in the dominant negative are not particularly limited, and include a kinase activity, a substrate-binding activity, and an intracellular localization activity. A dominant negative which lacks the function necessary to exert the effects of βARK1 interferes with a signaling pathway of βARK1. As a result, such a dominant negative functions as an inhibitor for βARK1. A typical example of a dominant negative for βARK1 is βARKct which is described in Examples of the present invention (Koch WJ *et al.*, Science 268: 1350-1353, 1995).

A βARK1 inhibitor of the present invention may also comprise a peptide fragment which is a substrate for βARK1. For example, a βARK1 inhibitor of the present invention may be a peptide fragment comprising amino acid position 56 to 74 or position 219 to 243 of the βAR amino acid sequence (US Patent No. 6096705). USP6096705 also has disclosed the use of high molecular weight compounds comprising a polyanionic structure such as heparin, dextran sulfate, polyaspartic acid, and polyglutamic acid.

A βARK1 inhibitor of the present invention may be a vector into which a DNA encoding a peptide or a protein comprising amino acids such as the dominant negatives and peptide fragments of βAR described above, has been inserted. Preferably, a DNA encoding a βARK1 inhibitor is operably linked to a promoter for an efficient expression. An exemplary promoter used for this purpose is CMV promoter. Amyocardial cell-specific promoter may also be used for tissue-specific expression of the DNA. Exemplary ventricular myocyte-specific promoters include ventricular myosin light chain 2 promoter and ventricular myosin heavy chain promoter.

The vectors as described above may also be used for gene therapy (Akhter SA *et al.*, Restoration of β-adrenergic signaling in failing cardiac ventricular myocytes via adenoviral-mediated gene transfer. Proc Natl Acad Sci USA 94: 12100-12105, 1997; Ashish SS *et al.*, In vivo ventricular gene delivery of a β-adrenergic receptor kinase inhibitor to the failing heart reserves cardiac dysfunction. Circulation 103: 1311-1316, 2001). Gene therapy refers to treatment or prevention of a disease by administering to a patient a vector comprising a DNA encoding a pharmaceutically effective protein. Exemplary vectors for use in gene therapy include, but are not limited to, adenoviral vectors (for example, pAdex1cw) and retroviral vectors (for example, pZIPneo). Ordinary genetic manipulations such as insertion of the DNA encoding βARKct in a vector may be conducted by a procedure normally used in the art. Such vectors can be administered to a living body by an *ex vivo* method, preferably an *in vivo* method.

A βARK1 inhibitor of the present invention may also include a substance obtained by screening test samples. Herein, test samples include, but are not limited to, single samples (e.g., a natural sample, an organic sample, an inorganic sample, a protein, and a peptide), sample libraries, expression products of a gene library, cell extracts, cell culture supernatants, products of a fermentative microorganism, extracts from a marine organism, and extracts from a plant.

βARK1 inhibitors may be screened by a method known to one skilled in the art, for example, a screening method using the phosphorylation activity of βARK1 as an index (Benovic J. L. Methods Enzymol 200: 351-362, 1991). A βARK1 inhibitor obtained by this screening method exhibits a βAR phosphorylation inhibitory activity of preferably, I-C₅₀ ≦ 10 nmol/L *in vitro*, and IC₅₀ ≦ 1 µmol/L in a cell system. The specificity of the βARK1 inhibitory activity may be measured by comparing the βARK1 inhibitory activity to an inhibitory activity for a commercially available kinase. IC₅₀ may be used to compare an enzyme inhibitory activity. SignaTECT^{TM} Protein Kinase Assay System (Promega) may be used with protein kinase A (PKA), protein kinase C (PKC), cdc2, DNA-dependent protein kinase, tyrosine kinase (PTK), and so on. A βARK1 inhibitor of the present invention preferably has a βARK1 inhibitory activity at least 10 times or higher, and more preferably at least 100 times or higher than the inhibitory activities for the above-mentioned five kinases.

As described above, βARK1 is a kinase whose substrate is βAR. It has also been revealed that rhodopsin is a substrate for βARK1 (Benovic J. L. *et al.*, Science 246: 235-240, 1989). Accordingly, rhodopsin may be used instead of βAR to assay the activity of βARK1. The source of rhodopsin used herein is preferably humans, but is not especially limited thereto. For example, bovine rhodopsin can be used. Rhodopsin may be prepared by a common procedure in the art, for example, the procedure as described below. Buffer A (10 mmol/L Tris-HCl, pH7.5, 65 mmol/L NaCl, and 2 mmol/L MgCl₂) containing 44% (w/v) of sucrose is added to retina in a dark place, preferably at low temperature and in a shade (under an infrared lamp). The resulting suspension is centrifuged at 25,000x g for 15 minutes to recover the supernatant. The precipitate is resuspended in buffer A containing 44% (w/v) sucrose, and the suspension is centrifuged to recover the supernatant. The supernatants are separately filtered and diluted with an equal volume of buffer A, and then centrifuged at 25,000xg for 40 minutes to collect the precipitate. The precipitate is suspended in buffer A containing 36% (w/v) sucrose, and centrifuged at 25,000x g for 30 minutes to collect the supernatant. The precipitate is suspended in buffer A containing 36% (w/v) sucrose, centrifuged, and the supernatant is collected. After diluting the supernatant with 1/2 volume of buffer A, the dilution is centrifuged at 25,000x g for 25 minutes to collect the precipitate. The precipitate thus recovered is suspended in buffer B (50 mmol/L Tris-HCl, pH7.5, 0.5 mmol/L EDTA, and 5 mol/L urea), and ground with a homogenizer. The homogenate is suspended in 50 mmol/L Tris-HCl (pH7.5) and centrifuged at 100, 000x g for 45 minutes to collect the precipitate.

When using rhodopsin as a substrate, a βARK1 inhibitory activity of a test sample may be easily measured *in vitro.* For example, a test sample solution in DMSO (preferably, at a final concentration of approximately 4 vol% DMSO) is added to 96-well Microwell, and a predetermined amount of the enzyme and the substrate (containing, at a final concentration, 3.1 µmol/L rhodopsin, 26.9 nmol/L purified βARK1, 20 mmol/L Tris-HCl (pH7.5), 2 mmol/L EDTA, and 5 mmol/L MgCl₂) is added at low temperature under an infrared lamp. After shaking for 5 minutes, [γ-³³P]ATP solution (containing ATP at a final concentration 40 µmol/L, [γ-³³P]ATP (16 kBq, Amersham Pharmacia Biotech, CAT# AH9968)) is added, and shaken for another 5 minutes. The reaction may be started by lighting such as room lighting. After 30 minutes, 25 vol% of trichloroacetic acid (TCA) /PBS is added, and the solution is shaken at a low temperature for 10 minutes to stop the reaction. The rhodopsin fraction is trapped on a filter plate by suction filtration, and the radioactivity is then measured by MicroBeta 1450 PLUS (Wallac) or other measuring equipment.

Another substrate for βARK1 than rhodopsin is the peptide RRREEEEESAAA (in one letter code for amino acids) described in a previous report (Chen CY *et al.* J Biol Chem 1993; 268: 7825-7831). For specific experimental procedure, one may refer to the procedure described in a previous report (Pitt MA *et al.* J Biomolecular Screening 1996; 1: 47-51).

βARK1 used for the screening is not particularly limited, and can be derived from, for example, human, bovine, hamster, or rabbit, and preferably human. βARK1 from human may be produced by genetic engineering. For example, primers can be designed based on the nucleotide sequence of the human βARK1 mRNA registered in GenBank (Accession No. M80776), and PCR amplification may be conducted by using a cDNA library from human leucocytes (GIBCO BRL, CAT# 10421-022) as a template to construct a plasmid. The recombinant human βARK1 protein can be expressed using the human βARK1 cDNA thus amplified and using an adequate combination of an expression vector and a host. A wide variety of expression vectors and hosts are commercially and readily available. For example, BacPAK Baculovirus Expression System (Clontech, Cat# K1601-1) can be used. In this expression system using an insect cell, homologous recombination takes place by co-transfection and a recombinant virus which expresses βARK1 in Sf-9 cells is constructed. When bovine βARK1 is to be used, the βARK1 can be purified by a method known in the art (for example, USP 6096705) .

In the screening using a cell system, cells in which a β2-adrenergic receptor is transiently expressed can be used. A β2-adrenergic receptor may be obtained either by purification from a natural source or genetic engineering. Genetic engineering is preferable because of a high product yield. A cDNA sequence of a β2-adrenergic receptor is registered in GenBank (AccessionNo. J02960).

A β2-adrenergic receptor may be transiently expressed by using a commercially available kit. For example, a β2-adrenergic receptor can be expressed in HEK293 cell by using FuGENE^{TM}6 Transfection Reagent (Roche CAT# 1 814 443). More specifically, cDNA of a β2-adrenergic receptor is transfected by using the kit described above, radiolabeled with Phosphorus-32 (Amersham Pharmacia Biotech, CAT# PBS13), and incubated at 37°C for 2 hours. The cells are then collected and suspended in 2 mL culture supernatant. After adding 0.2 mmol/L okadaic acid solution in DMSO (at final concentration of 100 nmol/L) and a test sample solution in DMSO (final DMSO concentration = 0.1 vol%), the cells are incubated at 37°C for 20 minutes. Next, (-) -isoproterenol (ISO) (SIGMA, CAT# 16504, at a final concentration of 10 µmol/L) may be added and incubated at 37°C for 15 minutes. After centrifuging twice with PBS, the precipiate was suspended in RIPA (+)buffer (50 mmol/L Tris-HCl (pH7.5), 150 mmol/L NaCl, 5 mmol/L EDTA, 1 vol% Nonidet P-40, 0.1% (w/v) SDS, 0.5% (w/v) sodium deoxycholate, 10 mmol/L NaF, 10 mmol/L disodium pyrophosphate, 0.1 mg/mL PMSF, and 1 tablet/50 mL complete^{TM} EDTA-free (Boehringer Mannheim, CAT# 1 873 580) ). The cells are then disrupted by passing the cells through a 26G needle five times to solubilize membrane proteins. After centrifuging at 15,000 rpm for 20 minutes, the supernatant is collected and 60 µL of Anti-HA Affinity Matrix (Roche, CAT# 1 815 016) is added for immunoprecipitation. After 24 hours, the precipitate is washed with 1 mL of RIPA (+) buffer, and 10 µL of RIPA (+) and 10 µL of 50 µg/mL HA peptide are added to elute at room temperature for 20 minutes. Five µL of 4x Sample buffer (8% (w/v) SDS, 400 mmol/L dithiothreitol, 240 mmol/L Tris-HCl (pH6.8), 40vol%glycerol, and 0.4mg/mL Bromophenol blue) is added and allowed to stand at room temperature for 10 minutes. After centrifugation, the supernatant is collected. Twenty µL of the supernatant is applied to 10% T SDS polyacrylamide gel and electrophoresed. The gel is then fixed on 3MM Paper. The amount of phosphorylation is quantitated with a measuring equipment such as BAS 2000 Image Analyzer (Fuji Photo Film Co., Ltd.).

The present invention also provides therapeutic agents for ischemic heart failure, which comprises a βARK1 inhibitor as described above as an active ingredient and which comprises an effect of improving the survival rate for ischemic heart failure. The therapeutic agents for ischemic heart failure may be formulated by a common procedure employed in the art with pharmaceutically acceptable vehicles as necessary. Exemplary vehicles include surfactants, excipients, colorants, flavoring agents, preservatives, stabilizers, buffer agents, suspending agents, isotonic agents, binders, disintegrants, lubricants, fluidity improving agents, and taste correctives. Such vehicles are not limited to those described above, and if necessary, other vehicles normally employed in the art may also be used. Specific examples thereof include light anhydrous silicic acid, lactose, microcrystalline cellulose, mannitol, starch, carmellose calcium, carmellose sodium, hydroxypropyl cellulose, hydroxypropyl methylcellulose, polyvinylacetal diethylaminoacetate, polyvinylpyrrolidone, gelatin, medium-chain triglyceride, polyoxyethylene hydrogenated castor oil, white sugar, carboxymethylcellulose, corn starch, and inorganic salts.

Therapeutic agents for ischemic heart failure as described above may be prepared into dosage forms such as oral agents, for example, tablets, powders, pills, epipastics, granules, fine granules, soft and hard capsules, film coated tablets, pellets, buccal tablets, and pastes, and parenteral agents, for example, injections, suppositories, percutaneous agents, ointments, plasters, and external liquids. The one skilled in the art would choose the most suitable dosage form according to administration route, target of administration, and so on.

The dosage of a therapeutic agent for ischemic heart failure of the present invention may be adequately determined by a physician considering the type of the dosage form, administration route, age and body weight of the patient, symptoms of the patient, type and seriousness of ischemic heart failure, and so on. An adult dosage for oral administration is generally 0.1 to 2000 mg per day and may be administered in one to several times per day. More preferably, the dosage is 1 to 1000 mg/day, still more preferably 50 to 500 mg/day, and most preferably 100 to 300 mg/day. While these dosages may vary depending on the body weight, age, administration route, and such, one skilled in the art can select an adequate dosage. Preferably, the administration period may also be determined according to the recovery process of the patient and so on.

This invention also provides a method for improving survival rate of a patient with ischemic heart failure, comprising administering to the patient an effective amount of a βARK1 inhibitor or a therapeutic agent for ischemic heart failure of the present invention to improve the heart function of the patient. In this method, pharmaceutical agents of the present invention formulated as described above may be administered by orally or parenterally.

### Brief Description of the Drawings

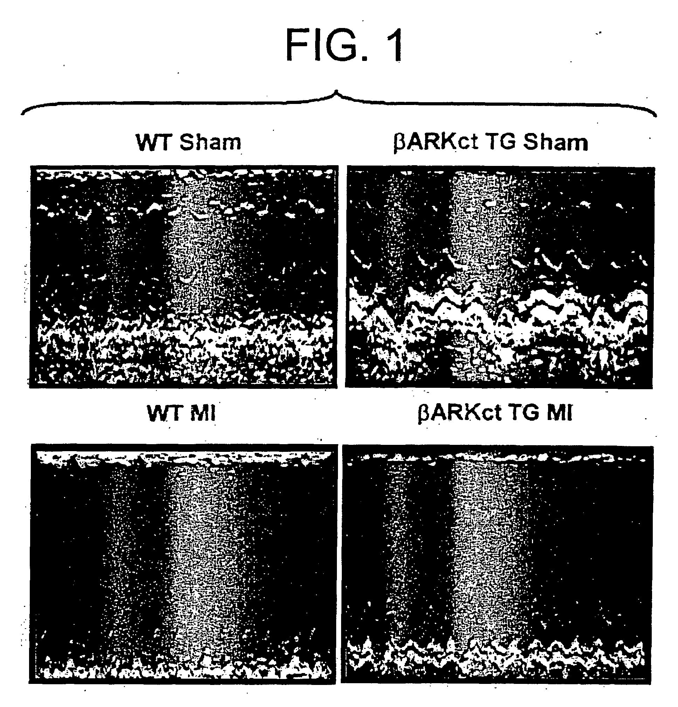

FIG. 1 shows representative left ventricular M-mode echocardiographic tracings for the myocardial infarction sham group of the litter mate control mice (WT-sham group), the myocardial infarction-produced group of the litter mate control mice (WT-MI group) , the myocardial infarction sham group of the βARKct TG mice (βARKct TG-sham group) , and the myocardial infarction-produced group of βARKct TG mice (βARKct TG-MI group).

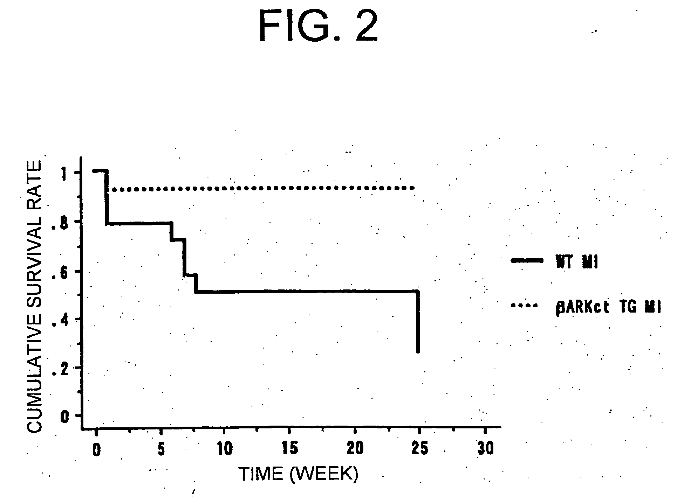

FIG. 2 shows survival curves for WT-MI group (n=14) and βARKct TG-MI group (n=11). The vertical axis represents cumulative survival rate, and the horizontal axis represents time (week) after the MI production surgery.

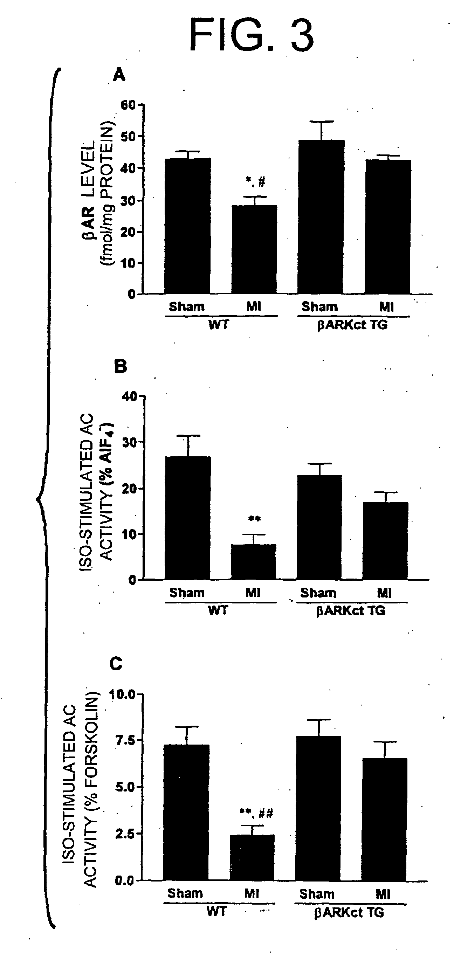

FIG. 3 shows graphs indicating βAR signaling profiles of WT-sham group, WT-MI group, βARKct TG-sham group, and βARKct TG-MI group. (A) βAR level in the hearts of WT mice and βARKct TGmice. (B) ISO-stimulated adenylyl cyclase (AC) activity corrected with AlF₄⁻-stimulated AC activity. (C) ISO-stimulated AC activity corrected with forskolin-stimulated AC activity. The bars in the histograms represent means ± standard error. For each group, n = 6. **, P<0.01; *, P<0.05 WT-MI vs. WT-Sham; ##, P<0.01; #, P<0.05 WT-MI vs. βARKct TG-MI (Tukey-Kramaer test).

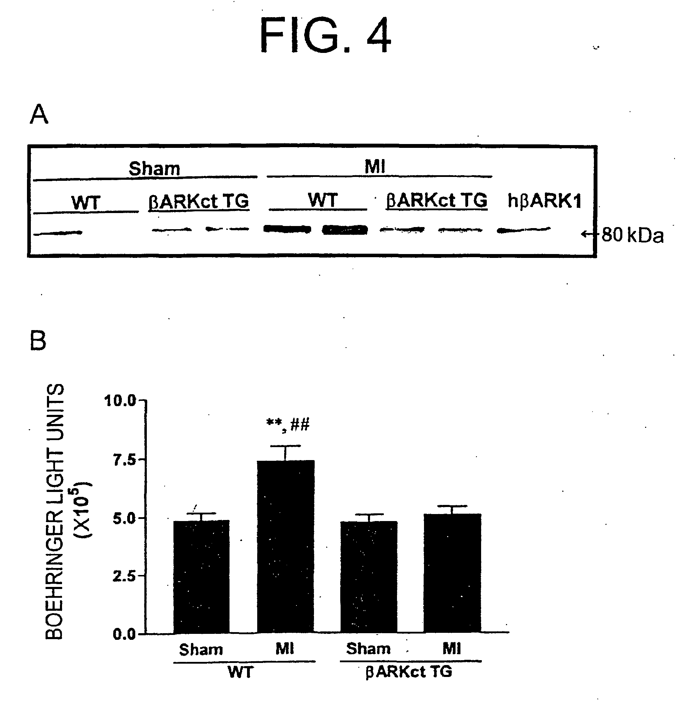

FIG. 4 shows a photograph and a graph showing the expression levels of the βARK1 protein in WT-sham group, WT-MI group, βARKct TG-sham group, and βARKct TG-MI group. (A) shows the results of immunoprecipitation analysis. hβARK1 represents the purified human ARK1. (B) shows the expression levels of the βARK1 protein in the hearts determined by the immunoblotting of βARK1. The bars in the histogram represent means ± standard error. For each group, n = 6. **, P < 0.01 WT-MI vs. WT-Sham; ##, P < 0.01 WT-MI vs. βARKct TG-MI (Tukey-Kramaer test).

### Best Mode for Carrying Out the Invention

Herein below, the present invention will be specifically described with reference to Examples, however, it is not to be construed as being limited thereto.

### [Example 1] Effects of a βARK1 inhibitor on the survival rate and cardiac functions of mice with cardiac failure after myocardial infarction

The materials and methods used for the experiment are described below in (1) and (2).

### (1) Experimental animals

Male βARKct TG (+/+) mice (B6, SJL-TgN (miniBARKct) 27Wjk, homozygotes) were purchased from Jackson Lab, self-bred, and mated with femaleC57BL/6Jmice (CLEAJapan, Inc.). The resultingmale βARKct TG (+/-) were mated with female C57BL/6J to obtain βARKct TG mice and litter mate controls (WT), which were used at11 to 13 weeks old.

PCR amplification was conducted by using the chromosomal DNA extracted from the tail of the mice as a template, and using a pair of primers set between αMHC promoter and β-globin gene inserted downstream of βARKct cDNA (sense strand, 5'- CTCCCCCATAAGAGTTTGAGTCG - 3' / SEQ ID NO: 1; and antisense strand, 5'- GGAACAAAGGAACCTTTAATAG - 3' / SEQ ID NO: 2). The mice whose DNA was amplified by the PCR (up to 800 bp) were determined to be TG, and the mice whose DNA was not amplified by the PCR were determined to be WT. The WT mice consisted of 26 females and 25 males, and the TG mice consisted of 30 females and 18 males.

### (2) Statistical analysis

The results are all shown as means ± standard error. Comparison of the survival rate was carried out by using Kaplan-Meier method and log-rank test (StatView ver.5 for Windows). Echocardiographic data were compared between the myocardial infarction sham group of the litter mate control mice (WT-sham group), the myocardial infarction-produced group of the litter mate control mice (WT-MI group), the myocardial infarction sham group of the βARKct TG mice βARKct TG-sham group), and the myocardial infarction-produced group of βARKct TG mice (βARKct TG-MI group), using multiple comparison of Turkey-Kramer (StatView ver.5 for Windows). The significance level was P < 0.05 (two sides).

### (3) Experimental procedure and results

Myocardial infarction (MI) was produced by using the WT and βARKct TG mice basically according to the procedure of Patten *et al.* (Patten RD *et al.*, Am J Physiol 274: H1812-H1820, 1998). The mice were anesthetized with 2.5% Avertin (prepared by dissolving 2,2,2-tribromoethanol [Cat. T4840-2, Aldrich] in tert-amyl alcohol [Cat. 24048-6, Aldrich] at 100 % (w/v), and diluting the solution with saline to 2.5 vol%) (14 µL/g, i.p.), and fixed in the supine position. An external cylinder of 22G indwelling needle was inserted from palate (for airway management), and artificial ventilation (1 mL/stroke, 120 cycles/min; model SN-480-7, Shinano Seisakusho) was performed. After opening the chest at left third intercostal space, a left coronary artery near the tip of the auricle of left atrium was ligated with 7-0 silk suture with a 6-mm round needle (Matsuda Ika). In the sham groups, the same surgical treatment was conducted except that the silk suture was ligated in the air. After closing of the chest, the mice were awaked and returned to the cages. Seventeen mice of the WT-MI group (n = 31) died within one week after the surgery, and 14 animals of the βARKct TG-MI group (n = 25, excluding one animal in which MI was not confirmed in the autopsy) died (survival rate: WT-MI group, 45%; βARKct TG-MI group, 44%; NS). It is known that mice die of acute myocardial infarction within 24 hours after the MI surgery, and that male mice may die from heart rupture in one week after MI. Therefore, in this experiment the mice that were alive after one week of post-surgery were used for the analysis of survival from chronic heart failure (It was also confirmed in the present experiment that the death within one week after the surgery was caused by heart rupture). In the sham groups, no death case was found throughout the experimental period for both the WT (n=15) and the βARKct TG (n = 13). As a consequence, 15 mice of the WT-sham group, 14 mice of the WT-MI group, 13 mice of the βARKct TG-sham group, and 11 mice of the βARKct TG-MI group were used in the following experiment.

At the 8^{th} week after the surgery, the mice were anesthetized with 2.5% Avertin (14 µL/g, i.p.), and fixed in the supine position. Echocardiography was performed for all surviving mice (WT-sham group, n = 15; WT-MI group, n = 7; βARKct TG-sham group, n = 13; and βARKct TG-MI group, n = 10) (using 13 MHz probe [EUP-C13H] ; EUB8000, Hitachi Medical Corporation) (FIG. 1). Compared with the sham groups, both the βARKct TG-MI group and the WT-MI group showed eccentric left ventricular hypertrophy by enlargement of the left ventricular cavity and substantially impaired cardiac functions. The βARKct TG-MI group exhibited remarkably improved cardiac function compared to the WT-MI group.

Averages of three measurements were calculated for each parameter (left ventricular end-diastolic dimension [LVEDD], left ventricular end-systolic diameter [LVESD], septal wall thickness [SEPth], posterior wall thickness [PWth], heart rate [HR], and fractional shortening [FS]), and to serve as the data. The survival rate was monitored up to the 26^{th} week after the surgery. The βARKct TG-MI group exhibited a remarkably improved survival rate compared to the WT-MI group (FIG. 2, log-rank test, P = 0.0101). The mice of the WT-sham group and the βARKct TG-sham group were all alive under the same conditions.

After monitoring the survival rate, the mice were euthanized and the hearts were removed. After thoroughly washing the hearts with saline, the center in the axial direction of the ventricle was sliced in transverse direction, and photographs of the cross section of each section was taken (COOLPLX 950, NIKON). Each slice was then separated into the left ventricle and the right ventricle, measured for wet weight, frozen with liquid nitrogen, and stored at -80°C. Using the photographs of myocardial slice, the infarct size was determined by calculating the ratio of the perimeter of the scar region to that of the left ventricle (Sigma Scan Pro ver. 4.01, SPSS Inc.). It was visually comfirmed that MI was produced for all death cases during the measurement of the survival rate. The heart weight, the infarct size, and the echocardiographic data for the βARKct TG mice and the WT mice at 7^{th} to 8^{th} week after the MI surgery are shown in Table 1.

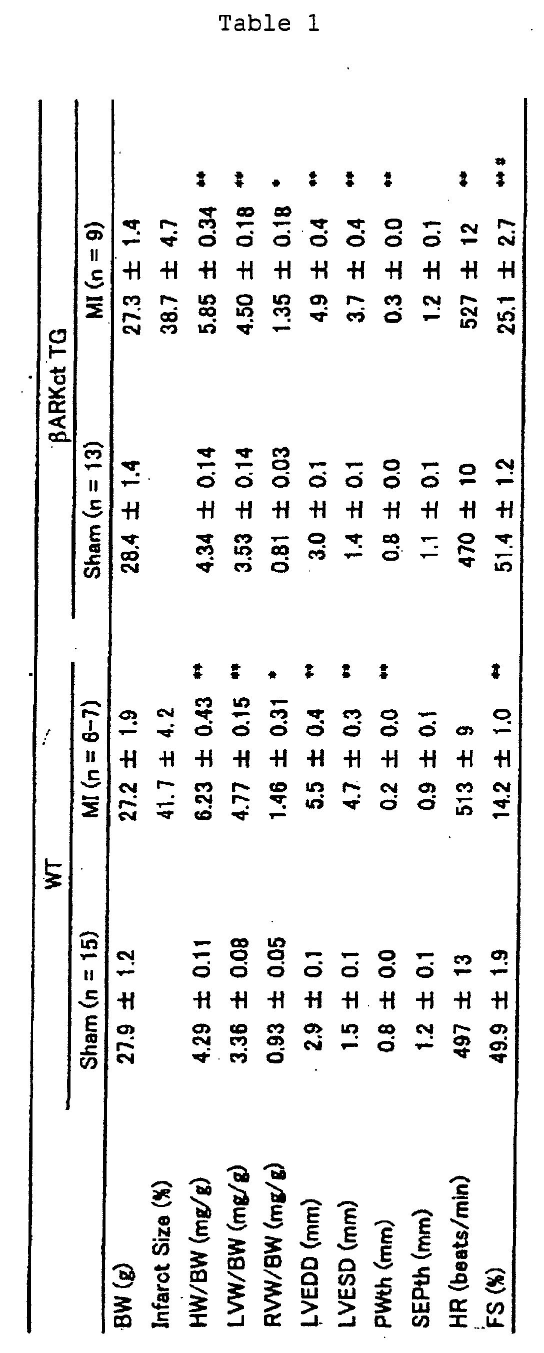

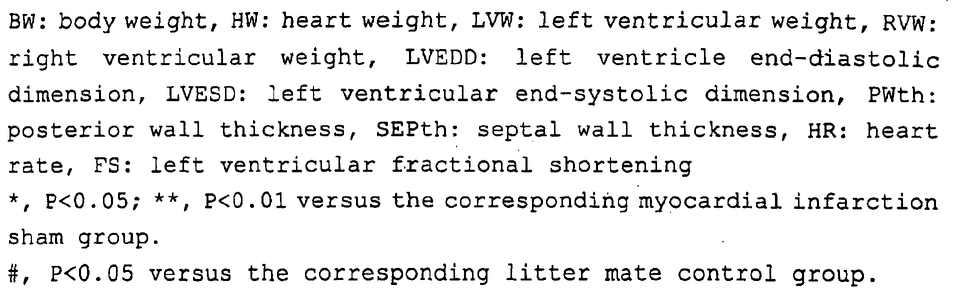

### [Example 2] Effects of a βARK1 inhibitor on a mouse model of heart failure after myocardial infarction

### (1) Methods

The hearts of the mice that had been frozen at -80°C were homogenized in an ice-cold lysis buffer (25 mmol/L Tris-HCl (pH 7.4), 5 mmol/L EDTA, 5 mmol/L EGTA, 10 µg/mL leupeptin, 20 µg/mL aprotinin, 1 mmol/L PMSF) (1 mL/100 g heart), and centrifuged at 500x g for 10 minutes. The supernatant was centrifuged at 15, 000x g for 15 minutes to separate into the membrane fraction and the cytosolic fraction. The membrane fraction was used for the measurement of the density of β-adrenergic receptor (βAR) and adenylyl cyclase (AC) activity. The cytosolic fraction was used to measure the amount of β-adrenergic receptor kinase 1 (βARK1) protein.

The membrane fraction (25 µg) was incubated with [¹²⁵I]cyanopindolol (NEX189, PerkinElmer) of various concentrations in a binding buffer (75 mmol/L Tris-HCl (pH 7.4), 12.5 mmol/L MgCl₂, 2 mmol/L EDTA) (200 µL) at 37°C for one hour. After suction filtration through GF/B glass filter, the fraction was washed, and a solid scintillator (Meltilex A, Wallac) was laid thereon, radioactivity was measured with MicoBeta (Wallac). The amount of specific binding was determined by subtracting the amount of non-specific binding (the amount of binding in the presence of 10 µmol/L propranolol) from the total amount of binding. The maximum amount of binding (Bmax.) was calculated using GraphPad PRISM (ver. 3.0) to serve as the βAR density.

The membrane fraction (20 µg) was incubated with 100 µmol/L isoproterenol (ISO)/50 µmol/L GTP, 30 mmol/L NaF/60 µmol/L AlCl₃, or 100 µmol/L forskolin (FSK) in a cyclase buffer (40 mmol/L Tris-HCl (pH 7.4), 10 mmol/L MgCl₂, 0.2 mmol/L EDTA, 100 mmol/L NaCl, 0.2 mmol/L dithiothreitol, 0.5 mmol/L ATP, 0.1 mmol/L 3-isobutyl-1-methylxanthine, 50 µg/mL phosphocreatine, and 5 IU/mL creatine phosphokinase) (100 µL) at 37°C for 10 minutes. The reaction was terminated by boiling at 95°C for 1.5 minutes, and centrifuged at 15,000x g for 15 minutes. The CAMP level in the resulting supernatant was then measured by cyclic AMP enzyme immunoassay system (RPN225, Amersham) . ISO, AlF₄⁻, and FSK-dependent AC activities were determined by subtracting the basal CAMP level from the measured CAMP level.

Thirty-five µL of agarose conjugated anti-GRK2 (βARK1) polyclonal antibody (sc-562AC, Santa Cruz) was added to one mL of the cytosolic fraction (3 mg/mL) in RIPA buffer (50 mmol/L Tris-HCl (pH 8.0), 5 mmol/L EDTA, 150 mmol/L NaCl, 1% Nonidet P-40, 0.5% sodium deoxycholate, 0.1% SDS, 10 mmol/L NaF, 5 mmol/L EGTA, 10 mmol/L sodium pyrophosophate, and 1 mmol/L PMSF) . The reaction mixture was immunoprecipitated at 4°C overnight, subjected to 12% SDS-PAGE, transferred onto a PVDF membrane. The amount of βARK1 protein (approximately 80 kDa) was determined using an anti-GRK2 polyclonal antibody (sc-562, Santa Cruz), an alkaline phosphatase conjugated anti-rabbit IgG, and CDP-Star (MS100R, TROPIX), measuring chemiluminescence with Lumi-Imager F1 (Boehringer Mannheim), and quantitating with Lumi-Analyst (ver. 3.0).

### (2) Statistical analysis

The results are shown as means ± standard error.

The data were compared by using multiple comparison of Turkey-Kramer (StatView ver.5 for Windows). Significance level was P<0.05 (two sides).

### (3) Results of the experiment

In the hearts of the wild-type (WT) mice with heart failure after myocardial infarction (MI), the PAR level was reduced by about 44%. On the other hand, such βAR level reduction was not found in the hearts of the post-MI βARKct transgenic (TG) mice (FIG. 3A). In the hearts of the post-MI WT mice, the ISO-stimulated AC activity decreased by about 71% (corrected with AlF₄⁻-stimulated AC activity) and by about 67% (corrected with forskolin-stimulated AC activity) while such a decrease in the ISO-stimulated AC activity was not found in the hearts of the post-MI βARKct TG mice (FIGs. 3B and 3C). Furthermore, no difference was found in the AlF₄⁻-stimulated AC activity and the forskolin-stimulated AC activity between the groups. In addition, in the hearts of the WT mice with post-MI heart failure, the βARK1 expression level was increased while no such change was found in the hearts of the post-MI βARKct TG mice (FIG. 4).

These results demonstrate that, while the levels of the GTP-binding protein and the AC activity were not reduced in the hearts with post-MI heart failure, the βARK1 expression level elevated, thereby causing the desensitization of βAR. In addition, it was revealed that the inhibition of βARK1 (the expression of βARKct) results in the recovery of the βARK1 expression level and the βAR signaling to the normal level.

### Industrial Applicability

As described above, this invention provides βARK1 inhibitors which have the effect of improving the survival rate in ischemic heart failure, and therapeutic agents for ischemic heart failure which comprise a βARK1 inhibitor as an active ingredient. The present invention enables the use of βARK1 inhibitors as efficacious therapeutic agents for ischemic heart failure, which have not only the cardiac function-improving effect but also the survival rate-improving effect, and it is highly expected to improve the poor prognosis of the patients with ischemic heart failure.

## Claims

1. An inhibitor for β adrenergic receptor kinase 1 (βARK1), which comprises an effect of improving the survival rate in ischemic heart failure.

2. A therapeutic agent for ischemic heart failure, , which comprises a βARK1 inhibitor as an active ingredient and comprises an effect of improving the survival rate in ischemic heart failure.

3. The therapeutic agent according to claim 2, wherein the ischemic heart failure is heart failure after myocardial infarction.

4. A method for improving the survival rate of a patient with ischemic heart failure, comprising administering to the patient the inhibitor of claim 1 or the therapeutic agent of claim 2 or 3 in an amount effective to improve the cardiac function of the patient.


SEQUENCE LISTING

<110> CHUGAI SEIYAKU KABUSHIKI KAISHA

<120> Remedy for ischemic heart failure

<130> C1-A0108P

<140>
<141>

<150> JP 2001-266489
<151> 2001-09-03

<160> 2

<170> PatentIn Ver. 2.1

<210> 1
<211> 23
<212> DNA
<213> Artificial Sequence

<220>
<223> Description of Artificial Sequence:an artificially
      synthesized primer sequence

<400> 1

ctcccccata agagtttgag tcg

<210> 2

<211> 22

<212> DNA

<213> Artificial Sequence

<220>

<223> Description of Artificial Sequence:an artificially

synthesized primer sequence

<400> 2

ggaacaaagg aacctttaat ag